# EUROPEAN PATENT SPECIFICATION

(11) **EP 2 537 511 B1**
(45) Date of publication and mention of the grant of the patent: **01.02.2017**
(21) Application number: 11171369.9
(22) Date of filing: 24.06.2011
(51) Int. Cl.: A61K 8/31, A61Q 9/02, A61K 8/37, A61K 8/49, A61K 8/81, B26B 21/44

(54) **Hair removal device comprising erodable moisturizer**
Haarentfernungsvorrichtung mit erodierbarer Feuchtigkeitscreme
Dispositif d'épilation comprenant un hydratant érodable

(43) Date of publication of application: 26.12.2012
(73) Proprietor: The Gillette Company LLC, Boston, MA 02127 (US)
(72) Inventor: Eagleton, Christopher, Raymond, Devizes, Wiltshire SN10 2FH (GB)
(74) Representative: Kohol, Sonia

(56) References cited:
- WO-A2-2009/114420
- GB-A- 2 423 494
- US-A1- 2008 060 201
- US-A1- 2010 011 588

## Description

### FIELD OF THE INVENTION

The present invention concerns the provision of a razor cartridge comprising a block of moisturizing material.

### BACKGROUND OF THE INVENTION

Hair removal devices incorporating a chemical composition are known and shall be referred to herein as devices comprising an "onboard" composition. Reference can be made to WO 07/056509 which teaches the inclusion of an onboard soap composition in a wet shaving razor. It is also known to provide a wet shaving razor incorporating an onboard skin-engaging composition comprising large quantities of hydrophilic polymers, such as polyethylene oxide, to lubricate the skin. Reference is made, by way of example, to WO 97/02116 and WO 97/02117. The patent applications referred to above relate to the provision of various advantages, such as additional lathering and soap-related benefits, or improved lubrication in the case of polyethylene oxide. It would alternatively or additionally be advantageous to be able to provide a skin moisturizing benefit via an onboard chemistry, especially to male users who may be less motivated to use skin moisturizers than females. The provision of a moisturizing benefit from an onboard chemistry may, however, have a number of difficulties associated with it.

Skin moisturization may be achieved in several different ways, but one important formulational route to achieving skin moisturization is to include materials which bind water, such as polyols. However, such materials derive their water-binding abilities in part from their significant hydrophilicity, which may render them unsuited for use in any context involving water, such as is the case during wet shaving - they may be washed away during the initial stages of a shave. An alternative approach might be to use occlusive, hydrophobic materials which cover the skin and therefore act to retain water already present therein. These materials are emollients which are less likely to be washed away during use in a highly aqueous environment. WO 06/108522, discloses the use of small amounts of hydrophobic emollients in an onboard chemistry. However, formulations comprising significant proportions of hydrophobic emollients may give rise to increased drag across the skin, due to the affinity of such materials with the hydrophobic skin surface. Users report that increased drag tends to increase discomfort during shaving. WO 2009/114420 A describes shaving aid materials, which can include lipophilic substances, as well as razor cartridges and razors including the shaving aid materials.

US 2009/0223057 discloses shaving aid strip compositions comprising polyoxyethylene for similar reasons to the above-discussed prior art. In order to improve the longevity of the shaving aid, the polyoxyethylene is mixed with amphipathic, but generally hydrophobic materials, such as fatty alcohols.

### SUMMARY OF THE INVENTION

According to a first aspect of the invention, a razor cartridge is provided comprising a housing having a base which is connectable to a handle, two or more blades, whose tips are aligned in a cutting plane to cut in a cutting direction, and a moisturizing block disposed before and/or after the blades in the cutting direction, the moisturizing block having a skin-contacting surface, wherein:
(a) the moisturizing block comprises at least 50% lipophilic materials by weight of the moisturizing block;
(b) the skin-contacting surface slopes away from the blades towards the base.

According to a second aspect of the invention, a razor is provided comprising a cartridge according to the first aspect of the invention.

According to a third aspect of the invention, the use of the razor according to the second aspect of the invention is provided, to remove hair and moisturize the skin.

### BRIEF DESCRIPTION OF THE DRAWINGS

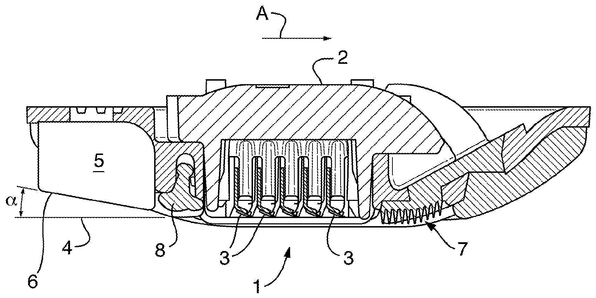

Fig.1. is a view of a razor cartridge according to the invention.

### DETAILED DESCRIPTION OF THE INVENTION

According to the invention, a razor cartridge is provided comprising at least two blades whose tips are aligned in a cutting plane to cut in a cutting direction. The razor cartridge comprises a housing having a base. The base is connectable to a handle via means known to the man skilled in this art and which need not be elaborated here.

The razor cartridge according to the invention comprises a moisturizing block. The moisturizing block is configured to be erodable, such that it erodes during and as a result of shaving human skin to leave moisturizing deposits on the skin. As used herein, the word "erodable", when used in relation to the moisturizing block, includes a moisturizing block having a Chatillon Hardness at 25°C of 0.50 - 3.25kg, preferably 0.75 - 3.00kg, more preferably 1.00 - 2.50kg, measured according to the protocol provided hereinbelow. Within these ranges, beneficial rates of wear may be achieved. In order to provide effective moisturization, the moisturizing block comprises at least 50%, preferably from 60% to 95% and more preferably from 70% to 90% lipophilic materials by weight of the moisturizing block.

The lipophilic materials may be liquid, semi-solid and/or solid at room temperature and may comprise one or more hydrocarbons, fatty acids, fatty alcohols, esters, triglycerides, fats, butters, waxes, lipophilic skin active agents or mixtures thereof.

Advantageously, if solids or semi-solids are present, then the moisturizing block comprises less than 20% and preferably less than 5% by weight of the moisturizing block of materials, and more preferably no materials at all, having a melting point of more than 100°C. This is because excessive quantities of such materials may render the moisturizing block inflexible and therefore liable to crack during manufacture and/or use.

Liquid, semi-solid, or solid hydrocarbon lipophilic materials which may be comprised within the moisturizing block include straight chain, branched chain, saturated and unsaturated hydrocarbons and mixtures thereof and they may comprise natural or synthetic hydrocarbon emollients and mixtures thereof. Preferred natural hydrocarbon emollients include petrolatum, mineral oil and mixtures thereof. Preferred synthetic hydrocarbon emollients include branched chain hydrocarbons, such as isohexadecane (such as Arlamol HD™ from Croda) and Polydecene (such as Puresyn 2™ from Exxon Mobil).

Liquid, semi-solid, or solid fatty alcohol or fatty acid emollients which may be comprised within the moisturizing block include saturated and unsaturated higher alcohols, especially C₁₂- C₃₀ fatty alcohols and fatty acids, especially lauric, myristic, palmitic, stearic, arachidic or behenic.

Liquid, semi-solid, or solid ester emollients which may be comprised within the moisturizing block include esters of a C₁₂ - C₃₀ alcohol and mixtures thereof, especially isopropyl myristate, isopropyl isostearate and mixtures thereof.

Liquid, semi-solid, or solid triglyceride emollients which may be comprised within the moisturizing block include synthetic or natural triglycerides, especially natural triglycerides derived from sunflower, avocado, olive, castor, coconut, cocoa and mixtures thereof. More preferred are coconut-derived triglycerides, such as the commercially available materials Myritol™ 312 and 318 (Cognis), Estasan™ (Croda) and Miglyol™ (Sasol).
Liquid, semi-solid, or solid fat and butter emollients which may be comprised within the moisturizing block include coconut butter, shea butter and mixtures thereof.

Liquid, semi-solid, or solid wax emollients which may be comprised within the moisturizing block include paraffin wax, microcrystalline wax, candellila, ozokerite and mixtures thereof, preferably paraffin wax. Advantageously, moisturizing block comprises some wax because waxes may bestow improved hardness and erodability to the moisturising block, although, as discussed above, the presence of too much wax may render the composition inflexible and therefore liable to crack during manufacture and/or use. Preferably, the moisturising block comprises from 2% to 20% and more preferably from 3% to 15% wax by weight of the moisturising block.
Liquid, semi-solid, or solid lipophilic skin active agents which may be comprised within the moisturizing block include oil soluble vitamins and agents which have activity on skin, such as vitamin E derivatives, including vitamin E acetate and tocopherol nicotinate; oil-soluble vitamin A derivatives, such as retinyl palmitate; lanolin; ceramides; sterols and sterol esters; salicylic acid; camphor; eucalyptol; essential oils and mixtures thereof.

The moisturizing block may be disposed before and/or after the blades in the cutting direction, but is preferably disposed after the blades. The moisturizing block comprises a skin-contacting surface which may be planar or may define a shallow convex and/or concave curve. Advantageously, the skin-contacting surface is planar, because a planar surface may provide a higher surface area for deposition than a convex or concave surface. The skin-contacting surface slopes away from the blades towards the base of the razor cartridge. This feature may reduce the drag experienced by users, while facilitating deposition of moisturiser by the moisturising block. Advantageously, the skin-contacting surface slopes such that all tangents to the skin-contacting surface slope at an acute angle, α, to the cutting plane. More advantageously, the angle, α, is from 5° to 15°.

The moisturizing block may additionally comprise a structuring polymer. Preferably, the moisturizing block comprises from 2% to 50%, preferably from 3% to 40%, more preferably 4% to 12% of structuring polymer by weight of the moisturizing block.

As defined herein, the structuring polymer is not regarded as being one of the "lipophilic materials" as defined above and should be ignored for the purposes of calculating the percentage weights of the "lipophilic materials".

Advantageously, the structuring polymer comprises a block copolymer. More advantageously, the block copolymer comprises a di-block copolymer, a tri-block copolymer, a multi-block copolymer, a radial block copolymer, a random block copolymer, or a mixture of these polymers.

In the case in which the block copolymer comprises a tri-block copolymer, then the tri-block copolymer preferably comprises a linear ABA tri-block polymer. Without wishing to be bound by theory, applicants believe that the A blocks aggregate creating domains, within which the moisturizing hydrophobic phase may accumulate, connected together by the B-blocks. This structure may provide an appropriate hardness to bestow the requisite wear properties to the moisturizing block, while also being flexible enough to be processed and not to crack or break during processing and/or use.

Advantageously, the linear ABA block copolymer comprises styrene-butadiene-styrene (SBS) block copolymer, styrene-isoprene-styrene (SIS) block copolymer, styrene-ethylenebutylene-styrene (S-EB-S) block copolymer, or mixtures thereof. More advantageously, the linear ABA block copolymer preferably comprises styrene-ethylenebutylene-styrene (S-EB-S) block copolymer. More advantageously still, the weight ratio of styrene to butadiene in the S-EB-S is in the range 20:80 to 40:60 and preferably around 30:70.

Particularly useful commercially available ABA block copolymers include Versagel™ materials available from Penreco and the Kraton™ G series, especially G-6150, G-1651, G-1652 and 1654. As discussed above, the structuring polymer comprised within the moisturizing block may comprise a random block copolymer. An example of a suitable random block copolymer is ethylene vinyl acetate (EVA) which is a copolymer of ethylene and vinyl acetate. Advantageously, the amount of ethylene comprised within the EVA polymer is from 65-90%, preferably from 70-85% by weight of the EVA to give beneficial wear properties. A commercially available range of EVA is called Elvax™, which is commercialised by DuPont. The moisturizing block may comprise one or more additional components which bestow a suitable melt viscosity to the composition, such as oil phase gellants, to facilitate improved processing, provided that the additional component(s) do not significantly reduce the hardness or erodability of the moisturizing block. Examples of such components are trihydroxystearin, which is commercially available as Thixcin R™ (manufactured by Elementis Specialities), ethylene vinyl acetate (EVA) and mixtures thereof.

A moisturizing block may be manufactured by heating the lipophilic materials to a suitable temperature to melt them, typically approximately 130°C, after which the structuring polymer is added and mixed well until the structuring polymer has dissolved. The mixture is then cooled, typically to approximately 90°C, after which any additional ingredients may be added. In a final step, the mixture is poured into suitable containers or moulds and allowed to cool to room temperature.
Once the mixture has set to form a moisturizing block, it may be affixed to a razor cartridge in any appropriate fashion. One such approach is to mould the moisturizing block directly onto the cartridge. Another approach involves directly or indirectly adhering the moisturizing block to the cartridge by means of an adhesive composition. One method of indirect adherence involves casting the moisturizing block onto a sheet of an appropriate substrate, such as an acetate sheet, which sheet is then adhered to the cartridge, for example mechanically or via an adhesive. Figure 1 depicts a non-limiting example of a razor cartridge (1) according to the invention. The cartridge (1) comprises a housing having a base (2) which is connectable to a handle using means known to the skilled person in this field. The cartridge (1) comprises blades (3) whose tips are aligned in a cutting plane (4) to cut in a cutting direction (A). The cartridge also comprises a moisturizing block (5). In this execution it is disposed after the blades (3), although it may instead or additionally be disposed before the blades (3). The moisturizing block (5) comprises skin-contacting surface (6), which slopes away from the blades (3) towards the base (2) at an angle, α, to the cutting plane (4). This embodiment also comprises a front skin-engaging element (7) and a rear skin-engaging element (8).

### Chatillon Hardness test

### Equipment: Chatillon TCD 200 equipped with a digital force gauge

### Sample preparation

1. Fully melt and cast moisturizing block into 60ml weigh boat (70mm X 70mm X 24mm)
2. Store lipid at 25°C overnight to equibrilate
3. Carefully remove moisturizing block from weigh boat prior to hardness testing

### Machine Preparation

A)
   1. Prepare Chatillon TCD 200 and digital force gauge according to manufacturers instructions.
   2. Set the ramp speed to 47 mm / min
B) Measuring the hardness value at 25°C:
   1. The pointed geometry should be attached to the shaft of ramp for this test method.
   2. Place the moisturizing block as prepared above and on its side onto the metal base plate directly below the centre of the shaft of the ramp. The mid-point of the moisturizing block should be in line with the centre of the shaft of the ramp.
   3. With the moisturizing block in place below the flat plate the speed set at 47 mm/min and the digital force gauge set at "C Peak" as above, depress the "Down" button on the Chatillon TCD200.
   4. Stop the Chatillon TCD200 just as the probe touches the surface of the moisturizing block and set the distance counter to zero.
   5. Reset the force gauge so that it reads zero
   6. Depress the "Down" button on the Chatillon TCD200 until the distance counter reads 13mm, record C Peak reading.

### Example

The following example discloses a moisturizing block which was incorporated into a razor cartridge as a strip disposed after the blade in the cutting direction. In use, when attached to a razor, the razor cartridge was observed to remove hair and moisturize the skin from which hair had been removed.

| **Trade Name** | **INCI Name** | **% w/w** |
|---|---|---|
| White soft paraffin | Petrolatum | 44.0 |
| Mineral oil | Paraffinum Liquidum | 44.0 |
| Kraton G1650E | Hydrogenated Styrene/Butadiene copolymer | 5.0 |
| Thixcin R | Trihydroxystearin | 2.0 |
| Paraffin Wax SP206 | Paraffin | 5.0 |

The composition of Example 1 was manufactured by heating the hydrocarbons and waxes to 130°C, then adding the linear ABA tri-block polymer (Kraton G1650E) and mixing well until polymer was fully dissolved. The mixture was then cooled to 90°C and the Thixcin added, after which the mixture was moulded onto the cartridge and allowed to cool to room temperature.

The Chatillon Hardness of the formulation of Example 1 is 1.7 and the angle, α, was moulded to be 12.4°.

## Claims

1. A razor cartridge (1) comprising a housing having a base (2) which is connectable to a handle, two or more blades (3), whose tips are aligned in a cutting plane (4) to cut in a cutting direction (A), and a moisturizing block (5) disposed before and/or after the blades in the cutting direction (A), the moisturizing block (5) having a skin-contacting surface (6), wherein:
(a) the moisturizing block (5) comprises at least 50% lipophilic materials by weight of the moisturizing block (5);
(b) the skin-contacting surface (6) slopes away from the blades (3) towards the base (2).

2. The razor cartridge (1) of claim 1, wherein the skin-contacting surface (6) is planar.

3. The razor cartridge (1) of claim 1, wherein the skin-contacting surface (6) defines a shallow convex or concave curve.

4. The razor cartridge (1) of claim 1 or 2, wherein all tangents to the skin-contacting surface (6) slope at an acute angle, α, to the cutting plane.

5. The razor cartridge (1) of claim 4, wherein the angle, α, is from 5° to 15°.

6. The razor cartridge (1) of any preceding claim, wherein the moisturizing block (5) comprises from 60% to 95%, preferably from 70% to 90% lipophilic materials by weight of the moisturizing block (5).

7. The razor cartridge (1) of any preceding claim, wherein the moisturizing block (5) comprises petrolatum, esters, triglycerides, waxes or mixtures thereof.

8. The razor cartridge (1) of any preceding claim, wherein the moisturizing block (5) comprises wax and preferably the moisturising block comprises from 2% to 20% and more preferably from 3% to 15% wax by weight of the moisturising block (5).

9. The razor cartridge (1) of any preceding claim, wherein the moisturizing block (5) has a Chatillon Hardness of 0.50kg to 3.50kg, preferably 0.75kg to 3.00kg, more preferably 1.00kg to 2.50kg.

10. The razor cartridge (1) of any preceding claim, wherein the moisturizing block (5) comprises from 2% to 50%, preferably from 3% to 40%, more preferably 4% to 12% of a structuring polymer by weight of the moisturizing block (5).

11. The razor cartridge (1) of claim 10, wherein the structuring polymer comprises a block copolymer.

12. The razor cartridge (1) of claim 11, wherein the block copolymer comprises a di-block copolymer, a tri-block copolymer, a multi-block copolymer, a radial block copolymer, a random block copolymer, or a mixtures of these polymers.

13. The razor cartridge (1) of any preceding claim, wherein the moisturizing block (5) is disposed after the cutting blades (3) in the cutting direction (A).

14. A razor comprising a cartridge (1) according to any preceding claim.

15. Use of a razor according to claim 14 to remove hair and moisturize the skin.

## Patentansprüche

1. Rasierklingeneinheit (1), umfassend ein Gehäuse mit einer Basis (2), die mit einem Griff verbunden werden kann, zwei oder mehr Klingen (3), deren Spitzen in einer Schneidebene (4) fluchten, um in einer Schneidrichtung (A) zu schneiden, und einen Befeuchtungsblock (5), der in der Schneidrichtung (A) vor und/oder hinter den Klingen angeordnet ist, wobei der Befeuchtungsblock (5) eine hautkontaktierende Oberfläche (6) aufweist, wobei:
(a) der Befeuchtungsblock (5) mindestens 50 % lipophile Materialien, bezogen auf das Gewicht des Befeuchtungsblocks (5), umfasst;
(b) die hautkontaktierende Oberfläche (6) von den Klingen (3) zu der Basis (2) hin abfällt.

2. Rasierklingeneinheit (1) nach Anspruch 1, wobei die hautkontaktierende Oberfläche (6) ebenflächig ist.

3. Rasierklingeneinheit (1) nach Anspruch 1, wobei die hautkontaktierende Oberfläche (6) eine flache konvexe oder konkave Kurve bestimmt.

4. Rasierklingeneinheit (1) nach Anspruch 1 oder 2, wobei alle Tangenten zu der hautkontaktierenden Oberfläche (6) in einem spitzen Winkel, α, zu der Schneidebene abfallen.

5. Rasierklingeneinheit (1) nach Anspruch 4, wobei der Winkel, α, von 5° bis 15° beträgt.

6. Rasierklingeneinheit (1) nach einem der vorstehenden Ansprüche, wobei der Befeuchtungsblock (5) zu 60 % bis 95 %, vorzugsweise zu 70 % bis 90 % lipophile Materialien, bezogen auf das Gewicht des Befeuchtungsblocks (5), umfasst.

7. Rasierklingeneinheit (1) nach einem der vorstehenden Ansprüche, wobei der Befeuchtungsblock (5) Petrolatum, Ester, Triglyceride, Wachse oder Mischungen davon umfasst.

8. Rasierklingeneinheit (1) nach einem der vorstehenden Ansprüche, wobei der Befeuchtungsblock (5) Wachs umfasst und der Befeuchtungsblock vorzugsweise zu 2 % bis 20 % und mehr bevorzugt zu 3 % bis 15 % Wachs, bezogen auf das Gewicht des Befeuchtungsblocks (5), umfasst.

9. Rasierklingeneinheit (1) nach einem der vorstehenden Ansprüche, wobei der Befeuchtungsblock (5) eine Chatillon-Härte von 0,50 kg bis 3,50 kg, vorzugsweise 0,75 kg bis 3,00 kg, mehr bevorzugt 1,00 kg bis 2,50 kg aufweist.

10. Rasierklingeneinheit (1) nach einem der vorstehenden Ansprüche, wobei der Befeuchtungsblock (5) zu 2 % bis 50 %, vorzugsweise zu 3 % bis 40 %, mehr bevorzugt zu 4 % bis 12 % ein Strukturierungspolymer, bezogen auf das Gewicht des Befeuchtungsblocks (5), umfasst.

11. Rasierklingeneinheit (1) nach Anspruch 10, wobei das Strukturierungspolymer ein Blockcopolymer umfasst.

12. Rasierklingeneinheit (1) nach Anspruch 11, wobei das Blockcopolymer ein Diblockcopolymer, ein Triblockcopolymer, ein Multiblockcopolymer, ein radiales Blockcopolymer, ein statistisches Blockcopolymer oder eine Mischung dieser Polymere umfasst.

13. Rasierklingeneinheit (1) nach einem der vorstehenden Ansprüche, wobei der Befeuchtungsblock (5) in der Schneidrichtung (A) hinter den Schneidklingen (3) angeordnet ist.

14. Rasierer, umfassend eine Rasierklingeneinheit (1) nach einem der vorstehenden Ansprüche.

15. Verwendung eines Rasierers nach Anspruch 14 zum Entfernen von Haar und Befeuchten der Haut.

## Revendications

1. Cartouche de rasoir (1) comprenant un boîtier ayant une base (2), qui peut être connectée à un manche, deux ou plusieurs lames (3) dont les pointes sont alignées dans un plan de coupe (4) afin de couper dans une direction de coupe (A) et un bloc d'hydratation (5) disposé avant et/ou après les lames dans la direction de coupe (A), le bloc d'hydratation (5) ayant une surface en contact avec la peau (6), dans laquelle
(a) le bloc d'hydratation (5) comprend au moins 50 % de matériaux lipophiles en poids du bloc d'hydratation (5) ;
(b) la surface en contact avec la peau (6) forme une pente en s'éloignant depuis les lames (3) vers la base (2).

2. Cartouche de rasoir (1) selon la revendication 1, dans laquelle la surface en contact avec la peau (6) est plane.

3. Cartouche de rasoir (1) selon la revendication 1, dans laquelle la surface en contact avec la peau (6) définit une courbe convexe ou concave superficielle.

4. Cartouche de rasoir (1) selon la revendication 1 ou 2, dans laquelle toutes les tangentes à la surface de contact avec la peau (6) forment une pente selon un angle aigu, α, par rapport au plan de coupe.

5. Cartouche de rasoir (1) selon la revendication 4, dans laquelle l'angle, α, va de 5° à 15°.

6. Cartouche de rasoir (1) selon l'une quelconque des revendications précédentes, dans laquelle le bloc d'hydratation (5) comprend de 60 % à 95 %, de préférence de 70 % à 90 %, de matériaux lipophiles en poids du bloc d'hydratation (5).

7. Cartouche de rasoir (1) selon l'une quelconque des revendications précédentes, dans laquelle le bloc d'hydratation (5) comprend du pétrolatum, des esters, des triglycérides, des cires ou leurs mélanges.

8. Cartouche de rasoir (1) selon l'une quelconque des revendications précédentes, dans laquelle le bloc d'hydratation (5) comprend de la cire et, de préférence, le bloc d'hydratation comprend de 2 % à 20 % et, plus préférablement, de 3 % à 15 % de cire en poids du bloc d'hydratation (5).

9. Cartouche de rasoir (1) selon l'une quelconque des revendications précédentes, dans laquelle le bloc d'hydratation (5) présente une dureté de Chatillon de 0,50 kg à 3,50 kg, de préférence de 0,75 kg à 3,00 kg, plus préférablement de 1,00 kg à 2,50 kg.

10. Cartouche de rasoir (1) selon l'une quelconque des revendications précédentes, dans laquelle le bloc d'hydratation (5) comprend de 2 % à 50 %, de préférence de 3 % à 40 %, plus préférablement de 4 % à 12 % d'un polymère structurant en poids du bloc d'hydratation (5).

11. Cartouche de rasoir (1) selon la revendication 10, dans laquelle le polymère structurant comprend un copolymère séquencé.

12. Cartouche de rasoir (1) selon la revendication 11, dans laquelle le copolymère séquencé comprend un copolymère diséquencé, un copolymère triséquencé, un copolymère multi-séquencé, un copolymère séquencé radial, un copolymère séquencé aléatoire ou un mélange de ces polymères.

13. Cartouche de rasoir (1) selon l'une quelconque des revendications précédentes, dans laquelle le bloc d'hydratation (5) est disposé après les lames de coupe (3) dans la direction de coupe (A).

14. Rasoir comprenant une cartouche (1) selon l'une quelconque des revendications précédentes.

15. Utilisation d'un rasoir selon la revendication 14 pour éliminer les poils et hydrater la peau.
